# EUROPEAN PATENT APPLICATION

(11) **EP 4 800 011 A1**
(43) Date of publication of application: **02.09.2026**
(21) Application number: 24957169.6
(22) Date of filing: 24.12.2024
(51) Int. Cl.: C07D 319/12

(54) **PREPARATION METHOD FOR LACTIDE, AND DIRECT LACTIDE AND DEPOLYMERIZED LACTIDE**

(30) Priority: 11.10.2024 CN 202411419140
(71) Applicant: Petrochina Shanghai Advanced Materials Research Institute Co. Ltd, Shanghai 201306 (CN); PetroChina Company Limited, Dongcheng District Beijing 100007 (CN)
(72) Inventor: SUN, Shuangyi, Beijing 100724 (CN); HAN, Chenming, Beijing 100724 (CN); XIA, Yongliang, Beijing 100724 (CN)
(74) Representative: IP TRUST SERVICES
(86) International application number: PCT/CN2024/141829
(87) International publication number: WO 2026/076834

(57) **Abstract**

The present disclosure provides a method for preparing lactide, and a direct lactide and a depolymerized lactide. The method comprises subjecting lactic acid to a prepolymerization reaction obtain a prepolymerized product; subjecting the prepolymerized product to a monomer removal treatment, wherein the component separated from the prepolymerized product after the monomer removal treatment comprises the direct lactide; and subjecting the remaining prepolymerized product after the monomer removal treatment to a depolymerization reaction to obtain the depolymerized lactide. The method of the present disclosure is simple in process and can obtain lactide with high optical purity without melt crystallization.

## Description

### TECHNICAL FIELD

The present disclosure relates to the technical field of organic chemical engineering, in particular to a method for preparing lactide, and a direct lactide and a depolymerized lactide.

### BACKGROUND

A biodegradable material refers to a polymer material that has excellent performance during use and can be rapidly degraded upon hydrolysis by enzymes or microorganisms after use. Among various biodegradable materials, polylactic acid (PLA) has attracted great attention due to its unique good biocompatibility, degradability and excellent processability.

There are mainly two methods for synthesizing polylactic acid. One method is direct polycondensation of lactic acid, and although the production process of this method is simple, due to the presence of impurities in the system and the reversible reaction of lactic acid polycondensation, the obtained polylactic acid has relatively small molecular mass and poor strength and has little practical value. In another method for synthesizing polylactic acid, lactic acid is first oligomerized and depolymerized to obtain a cyclic dimer, lactide, and then ring-opening polymerization is performed with lactide as a monomer to obtain polylactic acid. Products with high relative molecular mass up to one million can be obtained by this method, which becomes the main method for synthesizing polylactic acid.

For lactide that is a key intermediate in the synthesis of PLA, researches have been focused on the synthesis process and key equipment thereof in the industry. At present, during the industrial synthesis of lactide, polylactic acid with a certain degree of polymerization is generally used to carry out cracking and cyclization reactions in the presence of a catalyst to obtain a lactide product, as disclosed in for example US 5247058, US 5338822, US 5521278, and EP 98203427.4.

To prepare high quality PLA, it is important to use high purity lactide. As such, lactide needs to be purified to a high degree before it is eligible for ring-opening polymerization to produce high quality PLA. Known lactide purification processes typically comprise one or more integrated distillation, condensation and melt crystallization steps as in US 5521278, US 5357034 and US 5214159.

A racemization reaction means that chiral carbon atoms in original L-configuration are inverted to be in D-configuration, or chiral carbon atoms in original D-configuration are inverted to be in L-configuration. PLA on the market mainly uses L-lactic acid as a raw material, and the corresponding racemization is the inversion of L-configuration to D-configuration. There are two chiral carbon atoms in each molecule of lactide, and when both of the two chiral carbon atoms are not subjected to racemization and maintain the original L-configuration, the obtained lactide is L-lactide, which is the mainly desired product; when one of the two chiral carbon atoms is subjected to racemization, the obtained lactide is a meso-lactide (m-lactide), and the whole molecule is rendered optically inactive because the chirality of the two chiral carbons is the exact opposite, hence the name meso-lactide. During the oligomerization-depolymerization of the lactide synthesis process, a racemization reaction may occur to produce m-lactide. At present, even if optically pure L-lactic acid is used (it is usually required that the content of L-lactic acid is greater than 99.5%) as a raw material and lactide is used as an intermediate to produce PLA, it is still inevitable to produce a large amount of m-lactide during the production of lactide due to racemization. Currently, the typical applications involving L-lactide require L-lactide having a very high optical purity, and to obtain L-lactide with such a purity, it is necessary to separate m-lactide to a high degree.

Known methods for separating m-lactide generally use rectification to separate the m-lactide-rich stream and the L-lactide-rich stream. For example, in the method of CN105324164A, rectification is used for separation, the obtained raw material has an m-lactide content of 2.89% and a L-lactide content of 88.28%, a stream with an m-lactide content of 24.96% is obtained at the top of the column, and a stream with an m-lactide content of 2.5% is obtained at the sideline. However, since m-lactide and L-lactide have the same molecular weight and differ only in the chirality of a single carbon atom, the separation of these two is extremely difficult and the energy consumption is very high.

If the optical purity of lactide needs to be further improved, or in other words, the content of m-lactide needs to be reduced, melt crystallization is usually used. For example, in CN105324164A, a stream with an m-lactide content of 2.5% and an L-lactide content of 97.27% obtained by rectification is sent to a melt crystallization unit, and subjected to purification by falling film crystallization three times continuously, to finally obtain 99.83% of L-lactide and 0.14% of m-lactide.

In fact, crystallization is the only industrially practical method known to obtain lactide with high optical purity from a mixture of m-lactide and L-lactide, i.e., to obtain lactide with a L-lactide content of greater than 99% and a m-lactide content of less than 1%. The differences among existing technologies primarily lie in the use of different crystallizers and crystallization conditions. However, the crystallization approach suffers from high energy consumption and limited yield.

Therefore, it is necessary to develop a simpler and more efficient method for obtaining lactide with high optical purity.

### SUMMARY

In order to solve the above problems in the prior art, an object of the present disclosure is to provide a method for preparing lactide and a product thereof. The method for preparing the present disclosure is simple in process and can obtain lactide with high optical purity without melt crystallization.

In order to achieve the above object, according to a first aspect of the present disclosure, provided is a method for preparing lactide, comprising:
subjecting lactic acid to a prepolymerization reaction to obtain a prepolymerized product comprising a lactic acid oligomer and a first batch of lactide, wherein the first batch of lactide is denoted as a direct lactide;
subjecting the prepolymerized product to a monomer removal treatment;
separating a component comprising the direct lactide from the prepolymerized product after the monomer removal treatment, wherein the separated component is denoted as a monomer-removed component; and
subjecting the remaining component from the prepolymerized product after the monomer removal treatment, denoted as a residual component, to a depolymerization reaction to obtain a second batch of lactide, wherein the second batch of lactide is denoted as a depolymerized lactide.

In the present disclosure, it has been discovered for the first time that during the process of oligomerizing lactic acid into a lactic acid oligomer, a small amount of lactic acid molecules may undergo a dimerization cyclization reaction to directly obtain a small amount of lactide, i.e., the aforementioned direct lactide. Surprisingly, it has been found in the present disclosure that when optically pure lactic acid was used as the raw material, such as L-lactic acid with a content of >99.5% (it should be noted that the same applies when D-lactic acid is used as the raw material), the optical purity of the above direct lactide was often higher than that of lactide obtained through subsequent depolymerization step. In other words, the proportion of m-lactide (meso-lactide) in the direct lactide produced as a by-product during the lactic acid prepolymerization stage is relatively low.

Unfortunately, in a conventional synthesis process of lactide, the prepolymerized product of lactic acid containing this lactide with a high optical purity (i.e., the direct lactide) is sent to the depolymerization step. Since a large amount of lactide is generated in the depolymerization step and substantial racemization occurs during this depolymerization step, the resulting lactide is a lactide with low optical purity, and this lactide with low optical purity is collected in combination with the direct lactide, resulting in a final product with lower optical purity. In the prior art, the above direct lactide has not been valued or separated.

Another possible reason that such direct lactide has not been valued and separated is that lactide is typically removed from the prepolymerized product of lactic acid by vaporization under high temperature and high vacuum conditions, and the high temperature and high vacuum conditions are also conditions for depolymerization of the prepolymerized product of lactic acid. Therefore, the conventional temperature and pressure conditions may lead to continual production of new lactide due to ongoing depolymerization while removing the direct lactide, so that researchers are unable to pay particular attention to the direct lactide having a higher optical purity, and even if they are aware, there is no effective means to separate it.

Therefore, simply discovering that the direct lactide has high optical purity is not sufficient to obtain a lactide product with high optical purity, and there is a need in the prior art for a method to further effectively separate the direct lactide while simultaneously suppressing the depolymerization of lactic acid oligomer.

Through extensive and sophisticated experiments, the present disclosure demonstrates that a direct lactide can be effectively separated from the prepolymerized product of lactic acid without causing depolymerization of lactic acid oligomer. By this separation, the existing lactide monomer in the prepolymerized product of lactic acid is removed with little to no depolymerization of the oligomer, and therefore this step is referred to in the present disclosure as a monomer removal treatment. It should be particularly noted that the so-called monomer removal treatment of the present disclosure is to remove lactide as a polylactic acid monomer, but not lactic acid, as lactic acid is not directly used as a monomer in polylactic acid synthesis.

By controlling the source, the method for preparing the present disclosure overcomes the above defects in the prior art by preferentially separating the direct lactide with high optical purity.

Depolymerized lactide is a conventional lactide with lower optical purity in the prior art. The depolymerization process has been described in many patents, and thus will not be elaborated here. For information on equipment, depolymerization temperature and pressure control, reference may be made to relevant descriptions in known patent literature, such as CN112898266A in its Example 7.

In some preferred embodiments of the present disclosure, the monomer removal treatment is carried out at a temperature of 180-200°C and an absolute pressure of 500-3500 Pa. By controlling the temperature and pressure, the monomer removal treatment of the present disclosure does not undergo oligomer depolymerization and racemization while allowing the direct lactide to be removed. A series of experiments were conducted in the present disclosure, and the results showed that the temperature required for the depolymerization reaction (which is a chemical process and therefore involves the breaking and rejoining of chemical bonds) was higher. Therefore, it is preferable to limit the temperature of the monomer removal treatment within the above range, which is more favorable to the removal of direct lactide without the depolymerization and racemization of oligomers.

In some preferred embodiments of the present disclosure, the monomer removal treatment is carried out at a temperature of 185-195°C and an absolute pressure of 800-2500 Pa. A series of experiments were conducted in the present disclosure, and the results showed that, at a temperature above 195°C, depolymerization will occur significantly, so it is further preferable to limit the temperature of the monomer removal treatment to 185-195°C. However, on the other hand, the temperature of the monomer removal treatment cannot be too low, because the vapor pressure of lactide is small at lower temperatures, making it difficult to effectively remove lactide.

In some preferred embodiments of the present disclosure, the temperature for the prepolymerization reaction is not higher than 195°C. Preferably, the prepolymerization temperature is controlled within the above range, which is more advantageous to inhibit the m-lactide produced in the prepolymerization stage.

Further preferably, the temperature for the prepolymerization reaction is not higher than 190°C.

Further preferably, the temperature for the prepolymerization reaction is not higher than 185°C.

When the reaction temperature in the oligomerization stage is controlled at 190°C or below, the direct lactide contains little to no m-lactide (<1%). Even if the temperature of the prepolymerization stage is increased to 195°C, the content of m-lactide in the direct lactide is still less than 4%.

Lactic acid oligomers can be prepared according to well-known information, such as that in US5142023. The lactic acid oligomer used in the present disclosure is prepared by the steps of:
mixing L-lactic acid or D-lactic acid with a catalyst (e.g., stannous octoate) at a mass ratio of 100:0.1-1 (e.g., 100:0.2)) and purging with nitrogen;
reducing the pressure to 10-60 kPa (e.g., 20 kPa), gradually increasing the temperature to 100-195°C (preferably 180-195°C), and separating the distillate (primarily water); gradually reducing the pressure and increasing the temperature as required by extraction of the distillate, to obtain the lactic acid oligomer.

Other process conditions for pre-polymerizing lactic acid to obtain lactic acid oligomers are well known to those skilled in the art. In addition to the above temperature condition control, other preparation conditions can be easily designed by those skilled in the art according to common knowledge, and will not be elaborated here.

In some preferred embodiments of the present disclosure, the weight percentage of the monomer-removed component in the lactic acid oligomer is not higher than 12%.

Further preferably, the weight percentage of the monomer-removed component in the prepolymerized product is not higher than 10%.

Further preferably, the weight percentage of the monomer-removed component in the prepolymerized product is not higher than 8%.

Controlling the yield in the monomer removal stage is more favorable for avoiding depolymerization and obtaining the direct lactide with high optical purity.

In some preferred embodiments of the present disclosure, the weight percentage of the direct lactide in the prepolymerized product is not higher than 10%. Another key control factor of the present disclosure is the amount of direct lactide removed. If a high yield of the monomer removal step is pursued blindly, it may lead to depolymerization of the lactic acid oligomer to produce a lactide with low optical purity. In some application scenarios, the desired optical purity of the final lactide is 96%, so it is acceptable to have a small amount of lactide with low optical purity blended in the direct lactide.

Further preferably, the weight percentage of the direct lactide in the prepolymerized product is not higher than 8%.

Further preferably, the weight percentage of the direct lactide in the prepolymerized product is not higher than 6%.

In some preferred embodiments of the present disclosure, in the monomer-removed component, the percentage of m-lactide in the total lactide is not higher than 4%.

Further preferably, in the monomer-removed component, the weight percentage of m-lactide is not higher than 2%.

Further preferably, in the monomer-removed component, the weight percentage of m-lactide is not higher than 1%.

Controlling the m-lactide within the above range is more favorable for improving the optical strength of the product.

In actual production, water and lactic acid are usually also contained in the monomer-removed component. In some preferred embodiments of the present disclosure, the preparation method further comprises subjecting the prepolymerized product to a flash evaporation treatment prior to the monomer removal treatment, wherein the flash evaporation is conducted under the conditions of a temperature of 120-170°C and a pressure of 1-20 kPa with a duration of 1-60 s.

Further preferably, the flash evaporation is conducted under the conditions of a temperature of 160-170°Cand a pressure of 4-10 kPa with a duration of 1-15 s.

Generally, the prepolymerized product contains water and lactic acid, and thus flash evaporation is optionally carried out before the monomer removal to remove a part of the water and lactic acid, reducing the difficulty of subsequent separation and purification. The flash evaporation process of the present disclosure is carried out at a lower temperature and higher absolute pressure than in the monomer removal treatment, in order to selectively remove water and lactic acid without causing lactide to be removed. The flash evaporation is preferably set to take place within a short residence time, and if the residence time is too long, the lactic acid oligomer will continue to react to generate new water and cause the molecular weight of the lactic acid oligomer to increase.

Since additional equipment investment is required for the flash evaporation, and water, lactic acid and lactide are easily separated, flash evaporation may not be performed as the subsequent separation process allows.

It is readily understood by those skilled in the art that the monomer-removed component and the depolymerized lactide are both obtained in the form of a mixture. The mixture usually further comprises lactic acid, water and oligomers (mainly dimers and trimers). Therefore, in some preferred embodiments of the present disclosure, the monomer-removed component and the depolymerized lactide are purified separately to obtain the polymer-grade lactide.

Methods for purifying crude products such as the monomer-removed component and the depolymerized lactide have been fully discussed in the prior art, and may generally be purified by methods including rectification. In some cases, the purification may also be performed by recrystallization, melt crystallization, coupling separation, or the like. However, it is well known that these methods have the drawbacks of high energy consumption and limited yield. Therefore, in practical applications, purification is preferably carried out by rectification.

The direct lactide has a low monomer removal temperature, low removal ratio, and comprises relatively small amount of oligomers and larger amount of water and lactic acid, and therefore can be purified by water washing and drying. Such purification methods are widely known to those skilled in the art, and will not be elaborated here.

In some preferred embodiments of the present disclosure, the depolymerization reaction is carried out at a temperature of 195-240°C, and an absolute pressure of 133-13000 Pa.

Another unexpected discovery is that since the step of monomer removal treatment or flash evaporation and monomer removal treatment is performed prior to the conventional depolymerization process in the method for preparing the present disclosure, the content of water and lactic acid in the crude depolymerized lactide product obtained by the present disclosure is reduced, which further reduces the difficulty of subsequent purification (such as separation and purification by rectification).

According to another aspect of the present disclosure, a direct lactide obtained by the above preparation method is also provided.

In some preferred embodiments of the present disclosure, in the direct lactide, the mass percentage of m-lactide is ≤ 4%. Preferably, in the direct lactide, the mass percentage of m-lactide is ≤ 2%, further preferably ≤ 1%. The mass percentage of the m-lactide refers to m-lactide/(m-lactide + L-lactide + D-lactide), and lactic acid, water, oligomers and the like are excluded.

According to another aspect of the present disclosure, a depolymerized lactide obtained by the above preparation method is also provided.

Compared with the prior art, the present disclosure provides a new method for preparing lactide. By controlling the source from the start, this method avoids the conundrum where a lactide with high optical purity and a lactide with low optical purity are first mixed and then separated. The present disclosure can obtain lactide with high optical purity by using a relatively simple monomer removal treatment without the melt crystallization process. Moreover, the method provided by the present disclosure is advantageous in that it is simple in process and can utilize existing equipment, and has broad application prospects and enormous economic benefits.

### BRIEF DESCRIPTION OF THE DRAWINGS

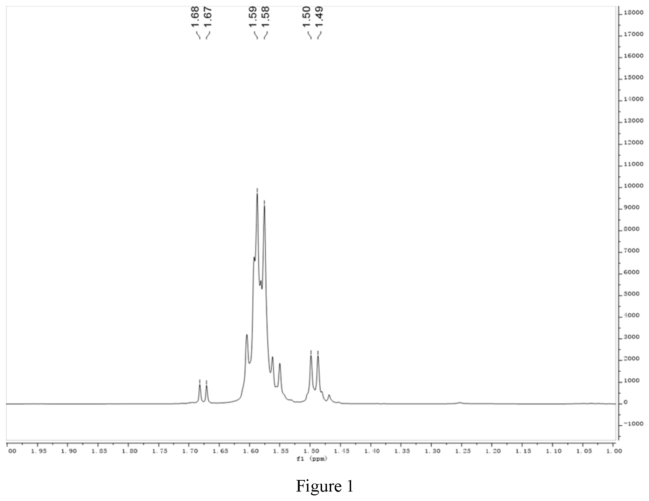

FIG. 1 shows the NMR spectrum of the lactic acid prepolymerized product 1 in Preparation Example 1.

### DESCRIPTION OF EMBODIMENTS

In order to have a clearer understanding of the technical features, objectives and beneficial effects of the present disclosure, the technical solutions of the present disclosure are now described in detail below, but should not be construed as limiting the implementable scope of the present disclosure.

In the following examples and comparative examples, the sources of some raw materials are as follows:
L-lactic acid, with an optical purity of greater than 99% and a chemical purity of greater than 99%, was purchased from Anhui Galactic Biochemical Co., Ltd.
D-lactic acid, with an optical purity of greater than 99% and a chemical purity of greater than 99%, was purchased from Sinopharm Chemical Reagent Co., Ltd.

Stannous octoate, as the catalyst, was purchased from Aladdin, Reagent grade.

Unless otherwise specified, other raw materials used in the examples or comparative examples were commercially available.

In the following examples and comparative examples, the solvent for nuclear magnetic resonance testing was deuterated chloroform.

The content percentages of L-lactide, D-lactide and m-lactide were determined by gas chromatography (GC) and calibrated using a standard curve. The chromatographic column and chromatographic conditions are shown in Table 1 below:

**Table 1**

| Chromatographic column | Capillary column |
|---|---|
| Stationary phase | Rt-βDEXsm |
| Column length (m) | 30 |
| Column inner diameter (mm) | 0.25 |
| Liquid film thickness (µm) | 0.25 |
| Carrier gas | Nitrogen |
| Carrier gas flow rate (mL/min) | 1 |
| Column temperature | 80°C hold for 2 min; heated to 150°C at 5°C/min, then heated to 220°C at 10°C/min, hold for 10 min |
| Gasification chamber temperature (°C) | 220 |
| Detector temperature (°C) | 230 |
| Injection volume (µL) | 1.0 |
| Split ratio | 30:01:00 |
| Hydrogen flow rate (mL/min) | 40 |
| Air flow rate (mL/min) | 400 |
| Purge gas flow rate (mL/min) | 30 |

When the main component is L-lactide, the optical purity = (L-lactide + m-lactide/2)/(L-lactide + m-lactide + D-lactide) × 100%.

When the main component is D-lactide, the optical purity = (D-lactide + m-lactide/2)/(L-lactide + m-lactide + D-lactide) × 100%.

Water content was titrated by the Karl Fischer method.

The residual amount of lactic acid in the fractions was determined by high performance liquid chromatography (HPLC), with chromatographic column: BEH C18 chromatographic column: (2.1 mm×100 mm×1.7 µm); mobile phase: phase A: phosphoric acid aqueous solution with a concentration of 0.02 mol/L, phase B: acetonitrile; mobile phase gradient: A: B = 60:40; chromatographic column flow rate: 0.15 ml/min, chromatographic column temperature: 40°C; ultraviolet detector wavelength: 210 nm; calibrated using a standard curve.

Unless otherwise specified, all percentages referred to below are calculated by mass.

### Preparation Example 1

This preparation example provides a method for preparing a lactic acid oligomer from lactic acid, comprising the following:
In a 5 L glass reactor equipped with a rectification column, a condenser and a fraction collection bottle, 3500 g of L-lactic acid and 7 g of stannous octoate were added, and purged with nitrogen for 3 minutes; the pressure was then reduced to 20 kPa, and the temperature was gradually increased. Starting at 95°C, liquid condensed in the condenser was observed, and the temperature was gradually and slowly increased to 190°C. Then, the pressure was reduced from 20 kPa to 10 kPa within 5 minutes, and maintained at 10 kPa for approximately 1 hour. A total of 620 g of fraction (mainly water) was collected, and a prepolymerized product 1 was obtained in the glass reactor.

The prepolymerized product 1 was sampled and tested by ¹H NMR, and the 1-2 ppm interval was shown in FIG. 1. As can be seen from FIG. 1, a double peak was observed at 1.67 ppm, which was confirmed to be L-lactide; no peak corresponding to m-lactide was clearly observed at 1.72 ppm.

### Preparation Example 2

This preparation example provides a method for preparing lactic acid oligomer from lactic acid, comprising the following:
In a 5 L glass reactor equipped with a rectification column, a condenser and a fraction collection bottle, 3500 g of D-lactic acid and 7 g of stannous octoate were added, and purged with nitrogen for 3 minutes; the pressure was then reduced to 20 kPa, and the temperature was gradually increased. Starting at 95°C, liquid condensed in the condenser was observed, and the temperature was gradually and slowly increased to 195°C. Then, the pressure was reduced from 20 kPa to 10 kPa within 5 minutes, and maintained at 10 kPa for approximately 1 hour. A total of 622 g of fraction (mainly water) was collected, and a prepolymerized product 2 was obtained in the glass reactor.

### Preparation Example 3

This preparation example provides a method for preparing lactic acid oligomer from lactic acid, comprising the following steps:
In a 5 L glass reactor equipped with a rectification column, a condenser and a fraction collection bottle, 3500 g of L-lactic acid and 7 g of stannous octoate were added, and purged with nitrogen for 3 minutes; the pressure was then reduced to 20 kPa, and the temperature was gradually increased. Starting at 95°C, liquid condensed in the condenser was observed, and the temperature was gradually and slowly increased to 180°C. Then, the pressure was reduced from 20 kPa to 10 kPa within 5 minutes, and maintained at 10 kPa for approximately 3 hours. A total of 618 g of fraction (mainly water) was collected, and a prepolymerized product 3 was obtained in the glass reactor.

### Example 1

This example provides a method for preparing lactide, comprising the following steps:
696.2 g of prepolymerized product 1 was placed in a 1 L glass bottle which was connected to a distillation head, a condenser and a collection bottle. Heat transfer oil circulating at 70°C was used in the condenser to control the temperature. The end of the collection bottle was connected to a vacuum pump.

The prepolymerized product 1 was first heated to 195°C, and then the pressure was rapidly reduced to 2.5 kPa, to start the monomer removal step, which took about 15 min. Until there was no obvious distillate in the condenser tube, 47.86 g of monomer-removed component was obtained, and the monomer-removed component was represented in the prepolymerized product 1 at a mass percentage of about 6.9% and denoted as fraction 1A.

Subsequently, the vacuum was terminated, nitrogen was replenished to normal pressure, and the collection bottle was replaced. The remaining prepolymerized product was further heated to 215°C, and the pressure was reduced to 2.5 kPa, to perform the depolymerization step. The depolymerization step was carried out for about 2 hours, until the temperature-controlled thermocouple in the bottle was about to leave the liquid level. It was observed that the residual contents in the bottle became yellow, and the viscosity was increased. 514.48 g of the depolymerized product obtained in the bottle was collected, with a mass percentage in the prepolymerized product 1 of about 73.9%, and was denoted as fraction 1B.

Fractions 1A and 1B were weighed and tested for components, respectively, and the results are shown in Table 2. The proportion of other components is obtained by subtracting the proportions of the four components including L-lactide from 100%.

An example of further purification is as follows:
The rectification device was a 1 L glass rectification column with an inner diameter of 25 mm, filled with glass spring packing, and a packing section height of 1.2 m. The rectification tower had heat tracing, with an initial heat tracing temperature of 120°C, and requires preheating. The condensing liquid in the condenser at the top of the column was ethylene glycol monomethyl ether. There were three alternating collection bottles at the top of the tower, each with a stopper to prevent cross-contamination.

512.2 g of fraction 1B was placed in a rectification device, and purged with nitrogen for 3 times. The temperature of the condensate at the top of the column was controlled at 25°C, with the vacuum degree controlled at 1.2 kPa and a reflux ratio for total extraction, and the bottom of the column was rapidly heated to about 100°C. During this period, about 9.1 g of fraction was distilled, which was denoted as rectified fraction 1 (presumably not completely condensed). The main components of rectification fraction 1 were water and lactic acid.

Subsequently, the bottom of the column was rapidly heated to 150°C, and the temperature of the condensing liquid at the top of the column was heated to 88°C, with a vacuum degree at 1.2 kPa and the reflux ratio of 1:1. 24.2 g of fraction was extracted, which was denoted as a rectified fraction 2 (presumably not completely condensed). The rectified fraction 2 was a mixture of m-lactide, L-lactide, water, and lactic acid.

Then, the reflux ratio was changed to 5:1, and 387.06 g of fraction was extracted, which was denoted as a rectified fraction 3. As determined by GC, this fraction contained 85.1% of L-lactide, 12.93% of m-lactide, and 1.0% of D-lactide. The content of lactic acid was lower than the limit of detection (0.01%). Water content was determined to be less than 100 ppm (100 ppm is the lower limit of equipment stability test results). The content of lactic acid in the rectified fraction 3 was difficult to determine accurately using chromatography. By using the acid value titration method, the total carboxyl content in the lactic acid and oligomers was determined at 80 mol/t. The first distillation was completed.

380 g of the rectified fraction 3 was reloaded into the rectification device, and purged with nitrogen for 3 times. Then, the bottom of the column was rapidly heated to 135°C, and the temperature of the condensing liquid at the top of the tower was heated to 88°C, with a vacuum degree of 1.0 kPa and a reflux ratio of 1:1. 14 g of fraction was extracted, which was denoted as a rectified fraction 4. The rectified fraction 4 was a mixture of m-lactide, L-lactide and trace amounts of lactic acid and oligomers.

After that, the reflux ratio was changed to 5:1, and 300 g of fraction was extracted, which was denoted as a rectified fraction 5. As determined by GC, this fraction contained 85.82% of L-lactide, 13.04% of m-lactide, and 1.01% of D-lactide. The oligomer content was estimated to be <0.2 wt% by subtraction. The total carboxyl content was determined to be 6.2 mol/t using the acid value titration method, and the moisture content was determined to be less than 100 ppm (100 ppm is the lower limit of equipment stability test results). The rectified fraction 5 satisfied the requirements for polymer-grade lactide.

### Example 2

This example provides a method for preparing lactide, comprising the following steps:
777.1 g of prepolymerized product 2 was placed in a 1 L glass bottle which was connected to a distillation head, a condenser and a collection bottle. Heat transfer oil circulating at 70°C was used in the condenser to control the temperature. The end of the collection bottle was connected to a vacuum pump.

The prepolymerized product 2 was first heated to 200°C, and then the pressure was rapidly reduced to 1.2 kPa, to start the monomer removal step, which took about 15 min. Until there was no obvious distillate in the condenser tube, 87.35 g of monomer-removed component was obtained, and the monomer-removed component was represented in the prepolymerized product 2 at a mass percentage of about 11.3% and denoted as fraction 2A.

Subsequently, the vacuum was terminated, nitrogen was replenished to normal pressure, and the collection bottle was replaced. The remaining prepolymerized product was further heated to 230°C, and the pressure was reduced to 8.6 kPa, to perform the depolymerization step, with the pressure being gradually reduced to 3.5 kPa. The depolymerization step was carried out for about 0.5 hours, until the temperature-controlled thermocouple in the bottle was about to leave the liquid level. It was observed that the residue in the bottle became yellow, and the viscosity was increased. 553.11 g of the depolymerized product obtained in the collection bottle was collected, with a mass percentage in the prepolymerized product 2 of about 71.6%, and was denoted as fraction 2B.

Fractions 2A and 2B were weighed and tested for components, respectively, and the results are shown in Table 3. The proportion of other components is obtained by subtracting the proportions of the four components including D-lactide from 100%.

### Example 3

This example provides a method for preparing lactide, comprising the following steps:
707.8 g of prepolymerized product 3 was placed in a 1 L glass bottle which was connected to a distillation head, a condenser and a collection bottle. Heat transfer oil circulating at 70°C was used in the condenser to control the temperature. The end of the collection bottle was connected to a vacuum pump.

The prepolymerized product 3 was first heated to 180°C, and then the pressure was rapidly reduced to 0.5 kPa, to start the monomer removal step, which took about 45 min. Until there was no obvious distillate in the condenser tube, 41.04 g of monomer-removed component was obtained, and the monomer-removed component was represented in the prepolymerized product 3 at a mass percentage of about 5.8% and was denoted as fraction 3A.

Subsequently, the vacuum was terminated, nitrogen was replenished to normal pressure, and the collection bottle was replaced. The remaining prepolymerized product was further heated to 195°C, and the pressure was reduced to 0.5 kPa, to perform the depolymerization step. The depolymerization step was carried out for about 3 hours, until the temperature-controlled thermocouple in the bottle was about to leave the liquid level. It was observed that the residue in the bottle became yellow, and the viscosity was increased. 512.46 g of the depolymerized product obtained in the collection bottle was collected, with a mass percentage in the prepolymerized product 3 of about 72.4%, and was denoted as fraction 3B.

Fractions 3A and 3B were weighed and tested for components, respectively, and the results are shown in Table 4. The proportion of other components is obtained by subtracting the proportions of the four components including L-lactide from 100%.

### Example 4

This example provides a method for preparing lactide, with a flash evaporation treatment before monomer removal, comprising the following steps:
968.4 g of prepolymerized product 3 was subjected to flash evaporation using a falling film evaporator. The feed temperature was 170°C, the main evaporator temperature was 170°C, and the pressure was 4.5 kPa. The scraper speed was 400 rpm, and the scraper area was 0.1 m². The residence time was about 10 s (by visual inspection). The temperature of the primary condensing liquid was 20°C, and the secondary condensing liquid was protected with liquid nitrogen. 18.5 g of a fraction was collected by primary condensation as a mixture of about 13.5 g lactic acid and 5 g water. The prepolymerized product after flash evaporation was used for subsequent monomer removal and depolymerization.
701 g of prepolymerized product 3 after the flash evaporation was placed in a 1 L glass bottle which was connected to a distillation head, a condenser and a collection bottle. Heat transfer oil circulating at 70°C was used in the condenser to control the temperature. The end of the collection bottle was connected to a vacuum pump.

The prepolymerized product 3 upon flash evaporation was first heated to 195°C, and then the pressure was rapidly reduced to 1.8 kPa, to start the monomer removal step, which took about 30 min, to obtain 44.35 g of a monomer-removed component. The monomer-removed component was represented in the prepolymerized product 3 at a mass percentage of about 6.3%, and was denoted as fraction 4A.

Subsequently, the vacuum was terminated, nitrogen was replenished to normal pressure, and the collection bottle was replaced. The remaining prepolymerized product was further heated to 205°C, and the pressure was reduced to 1.2 kPa, to perform the depolymerization step. The depolymerization step was carried out for about 2 hours, until the temperature-controlled thermocouple in the bottle was about to leave the liquid level. It was observed that the residue in the bottle became yellow, and the viscosity was increased. 517.86 g of the depolymerized product obtained in the collection bottle was collected, with a mass percentage in the prepolymerized product 3 of about 73.9%, and was denoted as fraction 4B.

Fractions 4A and 4B were weighed and tested for components, respectively, and the results are shown in Table 4. The proportion of other components is obtained by subtracting the proportions of the four components including L-lactide from 100%.

### Comparative Example 1

This comparative example provides a method for preparing lactide, comprising the following steps:
681.9 g of prepolymerized product 1 was placed in a 1 L glass bottle which was connected to a distillation head, a condenser and a collection bottle. Heat transfer oil circulating at 70°C was used in the condenser to control the temperature. The end of the collection bottle was connected to a vacuum pump.

In one step, the temperature of the prepolymerized product 1 was raised to 215°C, and the pressure was reduced to 2.5 kPa, to perform the depolymerization step. The depolymerization step was carried out for about 2 hours, until the temperature-controlled thermocouple in the bottle was about to leave the liquid level. It was observed that the residue in the bottle became yellow, and the viscosity was increased. 560.54 g of the depolymerized product was collected, with a mass percentage in the prepolymerized product 1 of about 82.2%, which was denoted as fraction 1C.

Fraction 1C was weighed and tested for components, and the results are shown in Table 2.

### Comparative Example 2

This comparative example provides a method for preparing lactide, comprising the following steps:
781.3 g of prepolymerized product 2 was placed in a 1 L glass bottle which was connected to a distillation head, a condenser and a collection bottle. Heat transfer oil circulating at 70°C was used in the condenser to control the temperature. The end of the collection bottle was connected to a vacuum pump.

In one step, the temperature of the prepolymerized product 2 was raised to 230°C, and the pressure was reduced to 8.6 kPa, to perform the depolymerization step, and the pressure was gradually reduced to 3.5 kPa. The depolymerization step was carried out for about 0.5 hours, until the temperature control thermocouple in the bottle was about to leave the liquid level. It was observed that the residue in the bottle became yellow, and the viscosity was increased. 635.22 g of the depolymerized product was collected, with a mass percentage in the prepolymerized product 2 of about 81.3%, and was denoted as fraction 2C.

Fraction 2C was weighed and tested for components, and the results are shown in Table 3.

### Comparative Example 3

This comparative example provides a method for preparing lactide, comprising the following steps:
693.7 g of prepolymerized product 3 was placed in a 1 L glass bottle which was connected to a distillation head, a condenser and a collection bottle. Heat transfer oil circulating at 70°C was used in the condenser to control the temperature. The end of the collection bottle was connected to a vacuum pump.

In one step, the temperature of the prepolymerized product 3 was raised to 195°C, and the pressure was reduced to 0.5 kPa, to perform the depolymerization step. The depolymerization step was carried out for about 4 hours, until the temperature-controlled thermocouple in the bottle was about to leave the liquid level. It was observed that the residue in the bottle became yellow, and the viscosity was increased. 550.0 g of the depolymerized product was collected, with a mass percentage in the prepolymerized product 3 of about 79.3%, and was denoted as fraction 3C.

Fraction 3C was weighed and tested for components, and the results are shown in Table 4.

**Table 2**

| Fractions | 1A | 1B | 1C |
|---|---|---|---|
| L-lactide, % | 73.23% | 80.31% | 79.75% |
| m-lactide, % | 1.15% | 12.45% | 11.33% |
| D-lactide, % | 0.00% | 0.94% | 0.88% |
| Lactic Acid, % | 13.36% | 1.60% | 2.60% |
| Water, % | 4.26% | 0.59% | 0.95% |
| Other components, % | 8.00% | 4.11% | 4.49% |
| Optical purity, % | 99.2% | 92.4% | 92.9% |
| Total mass (g) | 47.86 | 514.48 | 560.54 |

**Table 3**

| Fractions | 2A | 2B | 2C |
|---|---|---|---|
| D-lactide, % | 80.20% | 80.54% | 79.58% |
| m-lactide, % | 3.14% | 12.85% | 12.22% |
| L-lactide, % | 0.20% | 0.97% | 0.97% |
| Lactic Acid, % | 7.85% | 1.29% | 2.21% |
| Water, % | 2.95% | 0.48% | 0.82% |
| Other components, % | 5.66% | 3.87% | 4.20% |
| Optical purity, % | 97.9% | 92.2% | 92.4% |
| Total mass (g) | 87.35 | 553.11 | 635.22 |

**Table 4**

| Fractions | 3A | 3B | 3C | 4A | 4B |
|---|---|---|---|---|---|
| L-lactide, % | 67.09% | 79.76% | 80.67% | 87.33% | 80.94% |
| m-lactide, % | 0.47% | 12.36% | 11.25% | 0.83% | 12.50% |
| D-lactide, % | 0.00% | 0.94% | 0.86% | 0.02% | 0.95% |
| Lactic Acid, % | 16.66% | 1.86% | 2.20% | 5.69% | 1.33% |
| Water, % | 6.25% | 0.70% | 0.82% | 2.13% | 0.50% |
| Other components, % | 9.53% | 4.39% | 4.19% | 4.00% | 3.79% |
| Optical purity, % | 99.6% | 92.4% | 93.0% | 99.5% | 92.4% |
| Total mass (g) | 41.04 | 512.46 | 550.03 | 44.35 | 517.86 |
| Ratio of m-lactide to lactide, % | 6.1% | 15.6% | 10.0% | 3.4% | 7.5% |

It can be seen from the results in Table 2 that the lactide in fraction 1A has a high optical purity. The results in Table 3 and Table 4 are similar to those in Table 2.

It can be seen from the results in Table 2 that the contents of lactic acid and water in fraction 1B are less than those in fraction 1C, so fraction 1B is easier to be purified by rectification than fraction 1C. The results in Table 3 and Table 4 are similar to those in Table 2.

It can be seen from the results in Table 4 that the contents of lactic acid and water in fraction 4A after flash evaporation are significantly reduced compared with those in fraction 3A without flash evaporation, and that the contents of lactic acid and water in fraction 4B are significantly reduced compared with those in fraction 3B, indicating that adding the step of flash evaporation is beneficial to reducing the difficulty of subsequent rectification and separation.

The above embodiments are provided to help those skilled in the art to understand and apply the present disclosure. It is obvious that those skilled in the art can easily make various modifications to these embodiments and apply the general principles described herein to other embodiments without making creative effort. Therefore, the present disclosure is not limited to the above embodiments, and improvements and modifications made by those skilled in the art based on the present disclosure without departing from the scope of the present disclosure shall fall within the protection scope of the present disclosure.

## Claims

1. A method for preparing lactide, comprising:
subjecting lactic acid to a prepolymerization reaction to obtain a prepolymerized product comprising a lactic acid oligomer and a first batch of lactide, wherein the first batch of lactide is denoted as a direct lactide;
subjecting the prepolymerized product to a monomer removal treatment;
wherein the component separated from the prepolymerized product by the monomer removal treatment is denoted as a monomer-removed component which comprises the direct lactide; and
subjecting the remaining component of the prepolymerized product after the monomer removal treatment, denoted as a residual component, to a depolymerization reaction to obtain a second batch of lactide, wherein the second batch of lactide is denoted as a depolymerized lactide.

2. The method according to claim 1, wherein the monomer removal treatment is carried out at a temperature of 180-200°C and an absolute pressure of 500-3,500 Pa.

3. The method according to claim 2, wherein the monomer removal treatment is carried out at a temperature of 185-195°C and an absolute pressure of 800-2,500 Pa.

4. The method according to claim 1, wherein the temperature for the prepolymerization reaction is 195°C or below.

5. The method according to claim 1, wherein the monomer-removed component is 12% by weight or less based on the prepolymerized product.

6. The method according to claim 1, wherein the direct lactide is 10% by weight or less based on the prepolymerized product.

7. The method according to claim 1, wherein in the monomer-removed component, m-lactide is comprised at 4% or less with respect to the total lactide.

8. The method according to claim 1, further comprising, prior to the monomer removal treatment:
subjecting the prepolymerized product to a flash evaporation treatment at a temperature of 120-170°C and a pressure of 1-20 kPa for a duration of 1-60 s.

9. The method according to claim 1, wherein the depolymerization reaction is carried out at a temperature of 195-240°C and an absolute pressure of 133-13,000 Pa.

10. A direct lactide obtained by the method according to any one of claims 1 to 9.

11. The direct lactide according to claim 10, wherein m-lactide is comprised at 4% by weight or less in the direct lactide.

12. A depolymerized lactide obtained by the method according to any one of claims 1 to 9.

## Amended claims

### Amended claims under Art. 19.1 PCT

1. A method for preparing lactide, comprising:
subjecting lactic acid to a prepolymerization reaction to obtain a prepolymerized product comprising a lactic acid oligomer and a first batch of lactide, wherein the first batch of lactide is denoted as a direct lactide;
subjecting the prepolymerized product to a monomer removal treatment;
wherein the component separated from the prepolymerized product by the monomer removal treatment is denoted as a monomer-removed component which comprises the direct lactide; and
subjecting the remaining component of the prepolymerized product after the monomer removal treatment, denoted as a residual component, to a depolymerization reaction to obtain a second batch of lactide, wherein the second batch of lactide is denoted as a depolymerized lactide.

2. The method according to claim 1, wherein the lactic acid comprises L-lactic acid or D-lactic acid.

3. The method according to claim 2, wherein the optical purity of the lactic acid is greater than 99%.

4. The method according to claim 2, wherein the chemical purity of the lactic acid is greater than 99%.

5. The method according to claim 1, wherein the monomer removal treatment is carried out at a temperature of 180-200°C and an absolute pressure of 500-3,500 Pa.

6. The method according to claim 5, wherein the monomer removal treatment is carried out at a temperature of 185-195°C and an absolute pressure of 800-2,500 Pa.

7. The method according to claim 1, wherein the temperature for the prepolymerization reaction is 195°C or below.

8. The method according to claim 7, wherein the temperature for the prepolymerization reaction is 190°C or below.

9. The method according to claim 8, wherein the temperature for the prepolymerization reaction is 185°C or below.

10. The method according to claim 1, wherein the monomer-removed component is 12% by weight or less based on the prepolymerized product.

11. The method according to claim 10, wherein the monomer-removed component is 10% by weight or less based on the prepolymerized product.

12. The method according to claim 11, wherein the monomer-removed component is 8% by weight or less based on the prepolymerized product.

13. The method according to claim 1, wherein the direct lactide is 10% by weight or less based on the prepolymerized product.

14. The method according to claim 13, wherein the direct lactide is 8% by weight or less based on the prepolymerized product.

15. The method according to claim 14, wherein the direct lactide is 6% by weight or less based on the prepolymerized product.

16. The method according to claim 1, wherein in the monomer-removed component, m-lactide is comprised at 4% or less with respect to the total lactide.

17. The method according to claim 1, wherein in the monomer-removed component, m-lactide is comprised at 2% or less with respect to the total lactide.

18. The method according to claim 1, wherein in the monomer-removed component, m-lactide is comprised at 1% or less with respect to the total lactide.

19. The method according to claim 1, further comprising, prior to the monomer removal treatment:
subjecting the prepolymerized product to a flash evaporation treatment at a temperature of 120-170°C and a pressure of 1-20 kPa for a duration of 1-60 s.

20. The method according to claim 19, wherein the flash evaporation treatment is carried out at a temperature of 160-170°C and a pressure of 4-10 kPa for a duration of 1-15 s.

21. The method according to claim 1, wherein the depolymerization reaction is carried out at a temperature of 195-240°C and an absolute pressure of 133-13,000 Pa.

Statement under Art. 19.1 PCT
The applicant(s) amends the claims and the basis for the amendments in the originally filed specification are as follows:
Delete original claims 10-12.
Add three new claims based on the disclosure on page 6, lines 18-19 of the description, respectively defining the specific type of lactic acid, optical purity, and chemical purity..
Add two new claims based on the disclosure on page 4, lines 7-8 of the description, respectively defining the temperature range of the prepolymerization reaction.
Add two new claims based on the disclosure on page 4, lines 22-23 of the description, respectively defining the weight percentage range of the monomer-removed component relative to the prepolymerized product.
Add two new claims based on the disclosure on page 4, lines 29-30 of the description, respectively defining the weight percentage range of direct lactide relative to the prepolymerized product.
Add two new claims based on the disclosure on page 5, lines 2-3 of the description, respectively defining the percentage range of m-lactide in the total lactide in the monomer-removed component.
Add one new claim based on the disclosure on page 5, line 8 of the description, defining the flash evaporation treatment conditions.

The Opinion points out that original claims 10-12 lack inventiveness and novelty. In response, delete original claims 10-12, thereby overcoming the above issue. The applicant believes that the above amendments do not go beyond the scope of the disclosure contained in the original application documents.
